# EUROPEAN PATENT SPECIFICATION

(11) **EP 3 004 349 B1**
(45) Date of publication and mention of the grant of the patent: **28.03.2018**
(21) Application number: 14727479.9
(22) Date of filing: 28.05.2014
(51) Int. Cl.: C12N 15/10, C12N 15/82, C12N 15/85, C12N 15/90, A01K 67/00

(54) **A METHOD FOR PRODUCING PRECISE DNA CLEAVAGE USING CAS9 NICKASE ACTIVITY**
VERFAHREN ZUR HERSTELLUNG EINER PRÄZISEN DNA-SPALTUNG MITTELS CAS9-NICKASE-AKTIVITÄT
PROCÉDÉ VISANT À PRODUIRE UN CLIVAGE D'ADN PRÉCIS PAR L'ACTIVITÉ DE CAS9 NICKASE

(30) Priority: 29.05.2013 DK 201370295
(43) Date of publication of application: 13.04.2016
(73) Proprietor: Cellectis S.A., 75013 Paris (FR)
(72) Inventor: DUCHATEAU, Philippe, 91210 Draveil (FR); BERTONATI, Claudia, 75003 Paris (FR)
(74) Representative: Almond-Martin, Carol
(86) International application number: PCT/EP2014/061178
(87) International publication number: WO 2014/191518

(56) References cited:
- WO-A1-2013/141680
- WO-A1-2013/142578
- WO-A1-2014/065596
- WO-A1-2014/089290
- WO-A1-2014/093694
- WO-A1-2014/150624
- L. CONG ET AL: "Multiplex Genome Engineering Using CRISPR/Cas Systems", SCIENCE, vol. 339, no. 6121, 15 February 2013 (2013-02-15), pages 819-823, XP055067741, ISSN: 0036-8075, DOI: 10.1126/science.1231143 -& L. CONG ET AL: "Supplementary Material to : Multiplex Genome Engineering Using CRISPR/Cas Systems", SCIENCE, vol. 339, no. 6121, 3 January 2013 (2013-01-03), pages 1-25, XP055067744, ISSN: 0036-8075, DOI: 10.1126/science.1231143
- WEI CHUANXIAN ET AL: "TALEN or Cas9 - rapid, efficient and specific choices for genome modifications.", JOURNAL OF GENETICS AND GENOMICS = YI CHUAN XUE BAO 20 JUN 2013, vol. 40, no. 6, 12 April 2013 (2013-04-12) , pages 281-289, XP002718601, ISSN: 1673-8527
- LEI S. QI ET AL: "Repurposing CRISPR as an RNA-Guided Platform for Sequence-Specific Control of Gene Expression", CELL, vol. 152, no. 5, 1 February 2013 (2013-02-01), pages 1173-1183, XP055068548, ISSN: 0092-8674, DOI: 10.1016/j.cell.2013.02.022
- SEUNG WOO CHO ET AL: "Targeted genome engineering in human cells with the Cas9 RNA-guided endonuclease", NATURE BIOTECHNOLOGY, NATURE PUBLISHING GROUP, NEW YORK, NY, US, vol. 31, no. 3, 1 March 2013 (2013-03-01), pages 230-232, XP002699850, ISSN: 1087-0156, DOI: 10.1038/NBT.2507 [retrieved on 2013-01-29]
- JIANG WENYAN ET AL: "RNA-guided editing of bacterial genomes using CRISPR-Cas systems", NATURE BIOTECHNOLOGY,, vol. 31, no. 3, 1 March 2013 (2013-03-01), pages 233-239, XP002699849,
- WOONG Y HWANG ET AL: "Efficient genome editing in zebrafish using a CRISPR-Cas system", NATURE BIOTECHNOLOGY, vol. 31, no. 3, 29 January 2013 (2013-01-29), pages 227-229, XP055086625, ISSN: 1087-0156, DOI: 10.1038/nbt.2501 -& WOONG Y HWANG ET AL: "Supplementary Material to: Efficient genome editing in zebrafish using a CRISPR-Cas system", NATURE BIOTECHNOLOGY, vol. 31, no. 3, 29 January 2013 (2013-01-29), pages 1-21, XP002718602, ISSN: 1087-0156, DOI: 10.1038/nbt.2501
- J. E. DICARLO ET AL: "Genome engineering in Saccharomyces cerevisiae using CRISPR-Cas systems", NUCLEIC ACIDS RESEARCH, vol. 41, no. 7, 4 March 2013 (2013-03-04), pages 4336-4343, XP055086617, ISSN: 0305-1048, DOI: 10.1093/nar/gkt135
- MARTIN JINEK ET AL: "RNA-programmed genome editing in human cells", E-LIFE, SAM LTD, GB, vol. 2, 29 January 2013 (2013-01-29), pages e00471-1, XP002699851, ISSN: 2050-084X, DOI: 10.7554/ELIFE.00471 [retrieved on 2013-06-28]
- G. GASIUNAS ET AL: "Cas9-crRNA ribonucleoprotein complex mediates specific DNA cleavage for adaptive immunity in bacteria", PROCEEDINGS OF THE NATIONAL ACADEMY OF SCIENCES, vol. 109, no. 39, 25 September 2012 (2012-09-25), pages E2579-E2586, XP055068588, ISSN: 0027-8424, DOI: 10.1073/pnas.1208507109
- M. JINEK ET AL: "A Programmable Dual-RNA-Guided DNA Endonuclease in Adaptive Bacterial Immunity", SCIENCE, vol. 337, no. 6096, 17 August 2012 (2012-08-17), pages 816-821, XP055067740, ISSN: 0036-8075, DOI: 10.1126/science.1225829
- P. MALI ET AL: "RNA-Guided Human Genome Engineering via Cas9", SCIENCE, vol. 339, no. 6121, 3 January 2013 (2013-01-03), pages 823-826, XP55111247, ISSN: 0036-8075, DOI: 10.1126/science.1232033
- RAN F ANN ET AL: "Genome engineering using the CRISPR-Cas9 system", NATURE PROTOCOLS, NATURE PUBLISHING GROUP, GB, vol. 8, no. 11, 1 November 2013 (2013-11-01), pages 2281-2308, XP009174668, ISSN: 1750-2799
- RAN F ANN ET AL: "Double nicking by RNA-guided CRISPR Cas9 for enhanced genome editing specificity.", CELL 12 SEP 2013, vol. 154, no. 6, 12 September 2013 (2013-09-12), pages 1380-1389, XP002718603, ISSN: 1097-4172
- HSU PATRICK D ET AL: "DNA targeting specificity of RNA-guided Cas9 nucleases.", NATURE BIOTECHNOLOGY SEP 2013, vol. 31, no. 9, September 2013 (2013-09), pages 827-832, XP002718604, ISSN: 1546-1696 -& HSU PATRICK D ET AL: "Supplementary Information: DNA targeting specificity of RNA-guided Cas9 nucleases.", NATURE BIOTECHNOLOGY SEP 2013, vol. 31, no. 9, September 2013 (2013-09), pages 1-26, XP002718605, ISSN: 1546-1696
- MALI PRASHANT ET AL: "Cas9 as a versatile tool for engineering biology.", NATURE METHODS OCT 2013, vol. 10, no. 10, October 2013 (2013-10), pages 957-963, XP002718606, ISSN: 1548-7105

## Description

### FIELD OF THE INVENTION

The present invention relates to a method of genome engineering based on the type II CRISPR system. In particular, the invention relates to a method for precisely inducing a nucleic acid cleavage in a genetic sequence of interest and preventing off-site cleavage in a T-cell. The method is based on the use of nickase architectures of Cas9 and single or multiple crRNA(s) harboring two different targets lowering the risk of producing off-site cleavage. The present invention also relates to T-cells and therapeutic applications related to the method described here. Polypeptides, polynucleotides, vectors, compositions are also disclosed herein.

### BACKGROUND OF THE INVENTION

Site-specific nucleases are powerful reagents for specifically and efficiently targeting and modifying a DNA sequence within a complex genome. There are numerous applications of genome engineering by site-specific nucleases extending from basic research to bioindustrial applications and human therapeutics. Re-engineering a DNA-binding protein for this purpose has been mainly limited to the design and production of proteins such as the naturally occurring LADLIDADG homing endonucleases (LHE), artificial zinc finger proteins (ZFP), and Transcription Activator-Like Effectors nucleases (TALE-nucleases).

Recently, a new genome engineering tool has been developed based on the RNA-guided Cas9 nuclease (Gasiunas, Barrangou et al. 2012; Jinek, Chylinski et al. 2012) from the type II prokaryotic CRISPR (Clustered Regularly Interspaced Short palindromic Repeats) adaptive immune system. The CRISPR Associated (Cas) system was first discovered in bacteria and functions as a defense against foreign DNA, either viral or plasmid. So far three distinct bacterial CRISPR systems have been identified, termed type I, II and III. The Type II system is the basis for the current genome engineering technology available and is often simply referred to as CRISPR. The type II CRISPR/Cas loci are composed of an operon of genes encoding generally the proteins Cas9, Cas1, Cas2 and Csn2a, Csn2bor Cas4 (Chylinski, Le Rhun et al. 2013), a CRISPR array consisting of a leader sequence followed by identical repeats interspersed with unique genome-targeting spacers and a sequence encoding the *trans*-activating tracrRNA.

The development and applications of TALENs and CRISPR/Cas9-mediated genome editing technologies are briefly summarized by Wei Chuanxian et al. (Journal of Genetics and Genomics, 40 (201), 281-289).

CRISPR-mediated adaptative immunity proceeds in three distinct stages: acquisition of foreign DNA, CRISPR RNA (crRNA) biogenesis and target interference. (see review (Sorek, Lawrence et al. 2013)). First, the CRISPR/Cas machinery appears to target specific sequence for integration into the CRISPR locus. Sequences in foreign DNA selected for integration are called spacers and these sequences are often flanked by a short sequence motif, referred as the proto-spacer adjacent motif (PAM). crRNA biogenesis in type II systems is unique in that it requires a trans-activating crRNA (tracRNA). CRISPR locus is initially transcribed as long precursor crRNA (pre-crRNA) from a promoter sequence in the leader. Cas9 acts as a molecular anchor facilitating the base pairing of tracRNA with pre-cRNA for subsequent recognition and cleavage of pre-cRNA repeats by the host RNase III (Deltcheva, Chylinski et al. 2011). Following the processing events, tracrRNA remains paired to the crRNA and bound to the Cas9 protein. In this ternary complex, the dual tracrRNA:crRNA structure acts as guide RNA that directs the endonuclease Cas9 to the cognate target DNA (Jinek, Chylinski et al. 2012). Target recognition by the Cas9-tracrRNA:crRNA complex is initiated by scanning the invading DNA molecule for homology between the protospacer sequence in the target DNA and the spacer-derived sequence in the crRNA. In addition to the DNA protospacer-crRNA spacer complementarity, DNA targeting requires the presence of a short motif adjacent to the protospacer (protospacer adjacent motif - PAM). Following pairing between the dual-RNA and the protospacer sequence, Cas9 subsequently introduces a blunt double strand break 3 bases upstream of the PAM motif (Garneau, Dupuis et al. 2010).

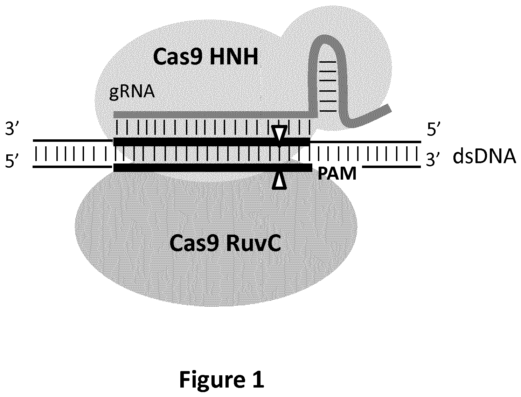

The large Cas9 protein (>1200 amino acids) contains two predicted nuclease domains, namely HNH (McrA-like) nuclease domain that is located in the middle of the protein and a splitted RuvC-like nuclease domain (RNase H fold) (Haft, Selengut et al. 2005; Makarova, Grishin et al. 2006). The HNH nuclease domain and the Ruv-C domain have been found to be essential for double strand cleavage activity. Mutations introduced in these domains have respectively led to Cas9 proteins displaying nickase-activity instead of double-strand cleavage activity. Different inactivating mutation(s) of the catalytic residues in the RuvC-like domains produces a nickase able to cut one strand in position +3bp (versus the 3' end) respect with the PAM location. The mutation of the catalytic residue of the HNH domain generates a nickase able to cut the other strand in position +3bp (versus the 5' end) (Jinek, Chylinski et al. 2012) (Figure 1).

Lei et al., (Cell, Vol. 152, n°5, February 2013) showed that a catalytically dead Cas9 lacking endonuclease activity, when co-expressed with a guide RNA, generated a DNA recognition complex that can specifically interfere with transcriptional elongation. RNA polymerase binding, or transcription factor binding. This system, designated CRISPR interference (CRISPRi), is reported to efficiently repress expression of targeted genes in *Escherichia coli.*

The CRISPR-Cas system has also been shown to function *in vivo* to induce targeted genetic modifications in zebrafish embryos with efficiencies similar to those obtained using zinc finger nucleases and transcription activator-like effector nucleases (Woong Y Hwang et al., Nature Biotechnology, Vol. 31, n°3, January 2013, p.227-229), and to provide robust and specific RNA-guided endonuclease activity at targeted endogenous genomic loci in *Saccharomyces cerevisiae* (Dicarlo et al., Nucleic Acid Research, Vol. 41, n°7, March 2013, p.4336-4343.

Jinek et al. (eLife, Vol. 2, January 2013, p.e00471) demonstrated the capacity of RNA-programmed Cas9 to introduce targeted double-strand breaks into human chromosomal DNA, thereby inducing site-specific genome editing reactions. Seung Woo Chung et al. (Nature Biotechnology, Vol. 31, n°3, March 2013) employed the CRISPR-Cas system for genome editing in human cells, showing that complexes of the Cas9 protein and artificial chimeric RNAs efficiently cleave two genomic sites and induce indels with frequencies of up to 33%.

Prokaryote type II CRISPR system is capable of recognizing any potential target sequence of 12 to 20 nucleotides followed by a specific PAM motif on its 3'end. However, the specificity for target recognition relies on only 12 nucleic acids (Jiang, Bikard et al. 2013; Qi, Larson et al. 2013), which is enough for ensuring unique cleavage site prokaryotic genomes on a statistical basis, but which is critical for larger genomes, like in eukaryotic cells, where 12 nucleic acids sequences may be found several times. There is therefore a need to develop strategies for improving specificity and reducing potential off-site using type II CRISPR system.

### SUMMARY OF THE INVENTION

Here the inventors have investigated different modifications into type II CRISPR system for improving specificity and reducing potential off-site cleavage in a T-cell. Unexpectedly, they found that using mutated version of Cas9 having nickase activity, instead of cleavase activity, can be used to produce cleavage at a given DNA target and increase the specificity in the same time. The method is based on the simultaneous use of nickase architecture of Cas9 (RuvC domain and/or HNH domain) and sgRNA(s) harboring two different complementary sequence to specific targets lowering the risk of producing off-site cleavage. By using at least one guide RNA harboring two different complementary sequence to specific targets or a combination of at least two guide RNA, the requirement for specificity passes from 12 to 24 nucleotides and, in turn, the probability to find two alternative binding sites of Cas9 (different from the ones coded in the two sgRNA) at an efficient distance from each other to produce an off-site cleavage gets really low. The invention extends to the crRNA, tracrRNA and Cas mutants designed to perform this method and to the T-cells transfected with the resulting modified type II CRISPR system.

### BRIEF DESCRIPTION OF THE FIGURES

**Figure 1****:** Schematic of the type II CRIPSR/Cas system mediated DNA double-strand break. In the type II CRISPR/Cas system, Cas 9 is guided by a two-RNA structure, named guide RNA (gRNA) formed by crRNA and tracRNA to cleave double-stranded nucleic acid target (dsDNA). Cas9 RuvC domain induces a nick event (arrow) in one strand in position +3bp (versus the 3'end) respect with the PAM location and the Cas9 HNH domain induces a nick event (arrow) in the other strand in position +3bp (versus the 5'end). For better understanding, the figure illustrates only one aspect of the CRISPR/Cas system mediated double-strand break.

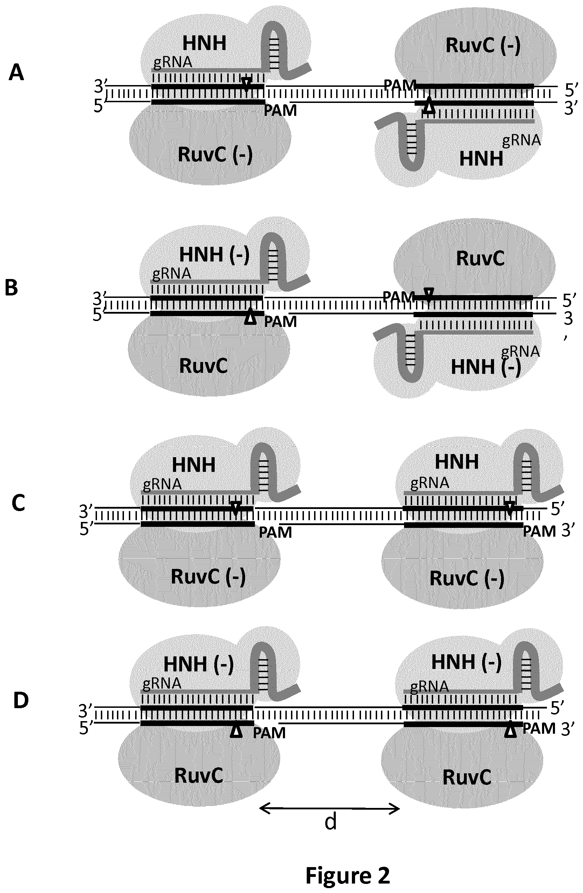

**Figure 2****:** Schematic of the new type II CRISPR/Cas system using a Cas9 nickase. **A-B** Two nucleic acid targets each comprising in one strand a PAM motif in the 3'-ends are selected within a genetic sequence of interest. The two nucleic acid targets are spaced by a distance "d". Cas9 harboring a non-functional RuvC or HNH domain (RuvC(-) (A-C) or (HNH(-) (B-D) respectively) is guided by two engineered gRNA each comprising a sequence complementary to at least 12 nucleotides adjacent to the complementary PAM motif of the first and second nucleic acid targets. **A-B.** Each PAM motifs of the two targets are present in different strands. The Cas9 nickase induces a nick (arrow) in the different strands resulting in a double-strand break within the genetic sequence of interest. **C-D.** Each PAM motifs of the two targets are present in the same strand. The Cas9 nickase induces a nick in the same strand of the genetic sequence of interest, resulting in the deletion of a single-strand nucleic acid sequence between the two nick events. The figure illustrates only some aspects of the CRISPR/Cas system using Cas9 nickase.

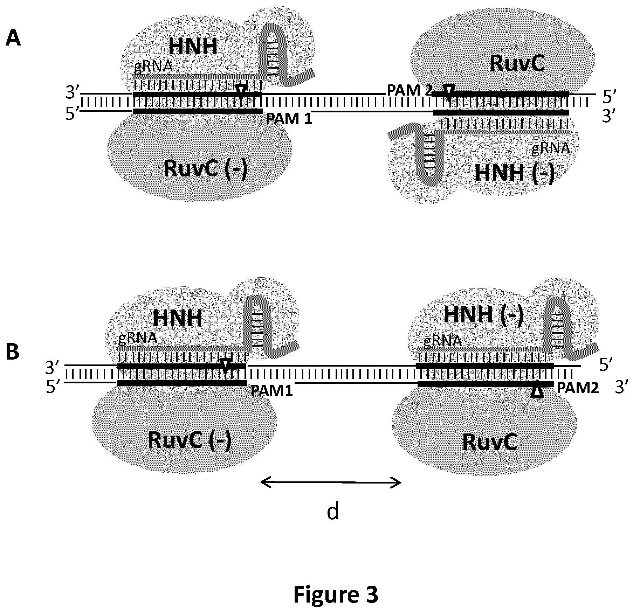

**Figure 3****:** Schematic of the new type II CRISPR/Cas system using two different Cas9 nickases. **A-B** Two nucleic acid targets comprising two different PAM motifs (PAM1 and PAM2) in the 3'end are selected within a genetic sequence of interest. The two nucleic acid targets are spaced by a distance "d". A first Cas9 harboring a non-functional RuvC is guided by an engineered gRNA which comprises sequence complementary to at least 12 nucleotides adjacent to the first complementary PAM motif. A second Cas9 harboring a non-functional HNH domain is guided by a second engineered gRNA which comprises a sequence complementary to at least 12 nucleotides adjacent to the second complementary PAM motif. **A.** Each PAM motifs of the two targets are present in the different strands. The two Cas9 nickases induce two nicks (arrows) in the same strand of the genetic sequence of interest, resulting in the deletion of a single-strand nucleic acid sequence between the two nick events. **B.** Each PAM motifs of the two targets are present in the same strand. The Cas9 nickases induce two nick events (arrows) in the different strands resulting in a double-strand break within the genetic sequence of interest. The figure illustrates only some aspects of the CRISPR/Cas system using Cas9 nickase.

### DISCLOSURE OF THE INVENTION

Unless specifically defined herein, all technical and scientific terms used have the same meaning as commonly understood by a skilled artisan in the fields of gene therapy, biochemistry, genetics, molecular biology and immunology.

All methods and materials similar or equivalent to those described herein can be used in the practice or testing of the present invention, with suitable methods and materials being described herein.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

### Method for precisely inducing a nucleic acid cleavage in a genetic sequence

The present invention thus relates to a new method based on the CRISPR/Cas system to precisely induce a cleavage in a double-stranded nucleic acid target in a T-cell. This method derives from the genome engineering CRISPR adaptive immune system tool that has been developed based on the RNA-guided Cas9 nuclease (Gasiunas, Barrangou et al. 2012; Jinek, Chylinski et al. 2012).

The present invention relates to a method for precisely inducing nucleic acid cleavage in a genetic sequence in a cell comprising:
(a) Selecting first and second double-stranded nucleic acid targets in said genetic sequence, each nucleic acid targets comprising, on one strand, a PAM motif at one 3' extremity;
(b) engineering two crRNAs comprising each:
   - a sequence complementary to one part of the opposite strand of the nucleic acid target that does not comprise the PAM motif, and
   - a 3' extension sequence ;
(c) providing at least one tracrRNA comprising a sequence complementary to one part of the 3' extension sequences of said crRNAs under b);
(d) providing at least one cas9 nickase which is a cas9 nuclease harboring either a non-functional RuvC-like or a non-functional HNH nuclease domain and specifically recognizing said PAM motif(s);
(e) introducing into the cell said crRNAs, said tracrRNA(s) and said Cas9 nickase; wherein the cell is a T-cell,
such that each Cas9-tracrRNA:crRNA complex induces a nick event in double-stranded nucleic acid targets in order to cleave the genetic sequence between said nucleic acid targets. Said cleavage can result from at least one nick event in one nucleic acid strand, preferably two nicks events in the same nucleic acid strand or more preferably two nick events on the opposite nucleic acid strands. Cas9, also named Csn1 (COG3513 - SEQ ID NO: 1) is a large protein that participates in both crRNA biogenesis and in the destruction of invading DNA. Cas9 has been described in different bacterial species such as *S. thermophilus* (Sapranauskas, Gasiunas et al. 2011), *listeria innocua* (Gasiunas, Barrangou et al. 2012; Jinek, Chylinski et al. 2012) and *S. Pyogenes* (Deltcheva, Chylinski et al. 2011). The large Cas9 protein (>1200 amino acids) contains two predicted nuclease domains, namely HNH (McrA-like) nuclease domain that is located in the middle of the protein and a splitted RuvC-like nuclease domain (RNase H fold) (Haft, Selengut et al. 2005; Makarova, Grishin et al. 2006).

HNH motif is characteristic of many nucleases that act on double-stranded DNA including colicins, restriction enzymes and homing endonucleases. The domain HNH (SMART ID: SM00507, SCOP nomenclature:HNH family) is associated with a range of DNA binding proteins, performing a variety of binding and cutting functions (Gorbalenya 1994; Shub, Goodrich-Blair et al. 1994). Several of the proteins are hypothetical or putative proteins of no well-defined function. The ones with known function are involved in a range of cellular processes including bacterial toxicity, homing functions in groups I and II introns and inteins, recombination, developmentally controlled DNA rearrangement, phage packaging, and restriction endonuclease activity (Dalgaard, Klar et al. 1997). These proteins are found in viruses, archaebacteria, eubacteria, and eukaryotes. Interestingly, as with the LAGLI-DADG and the GIY-YIG motifs, the HNH motif is often associated with endonuclease domains of self-propagating elements like inteins, Group I, and Group II introns (Gorbalenya 1994; Dalgaard, Klar et al. 1997). The HNH domain can be characterized by the presence of a conserved Asp/His residue flanked by conserved His (amino-terminal) and His/Asp/Glu (carboxy-terminal) residues at some distance. A substantial number of these proteins can also have a CX2C motif on either side of the central Asp/His residue. Structurally, the HNH motif appears as a central hairpin of twisted β-strands, which are flanked on each side by an α helix (Kleanthous, Kuhlmann et al. 1999). The other CRISPR catalytic domain RuvC like RNaseH (also named RuvC) is found in proteins that show wide spectra of nucleolytic functions, acting both on RNA and DNA (RNaseH, RuvC, DNA transposases and retroviral integrases and PIWI domain of Argonaut proteins).

Recently, it has been demonstrated that HNH domain is responsible for nicking of one strand of the target double-stranded DNA and the RuvC-like RNaseH fold domain is involved in nicking of the other strand (comprising the PAM motif) of the double-stranded nucleic acid target (Jinek, Chylinski et al. 2012). However, in wild-type Cas9, these two domains result in blunt cleavage of the invasive DNA within the same target sequence (proto-spacer) in the immediate vicinity of the PAM (Jinek, Chylinski et al. 2012). In the present invention, Cas 9 is a nickase and induces a nick event within different target sequences. As non-limiting example, Cas9 can comprise mutation(s) in the catalytic residues of either the HNH or RuvC-like domains, to induce a nick event within different target sequences. As non-limiting example, the catalytic residues of the compact Cas9 protein are those corresponding to amino acids D10, D31, H840, H868, N882 and N891 of SEQ ID NO: 1 or aligned positions using CLUSTALW method on homologues of Cas Family members. Any of these residues can be replaced by any other amino acids, preferably by alanine residue. Mutation in the catalytic residues means either substitution by another amino acids, or deletion or addition of amino acids that induce the inactivation of at least one of the catalytic domain of cas9. (cf (Sapranauskas, Gasiunas et al. 2011; Jinek, Chylinski et al. 2012). In a particular embodiment, Cas9 may comprise one or several of the above mutations. In another particular embodiment, Cas9 may comprise only one of the two RuvC and HNH catalytic domains. In the present invention, Cas9 of different species, Cas9 homologues, Cas9 engineered and functional variant thereof can be used. The invention envisions the use of such Cas9 variants to perform nucleic acid cleavage in a genetic sequence of interest. Said Cas9 variants have an amino acid sequence sharing at least 70 %, preferably at least 80 %, more preferably at least 90%, and even more preferably 95 % identity with Cas9 of different species, Cas9 homologues, Cas9 engineered and functional variant thereof. Preferably, said Cas9 variants have an amino acid sequence sharing at least 70 %, preferably at least 80 %, more preferably at least 90%, and even more preferably 95 % identity with SEQ ID NO: 1.

The nucleic acid cleavages caused by site-specific nucleases are commonly repaired through the distinct mechanisms of homologous recombination or non-homologous end joining (NHEJ). Although homologous recombination typically uses the sister chromatid of the damaged DNA as an exogenous nucleic acid sequence from which to perform perfect repair of the genetic lesion, NHEJ is an imperfect repair process that often results in changes to the DNA sequence at the site of the cleavage. Mechanisms involve rejoining of what remains of the two DNA ends through direct religation (Critchlow and Jackson 1998) or via the so-called microhomology-mediated end joining (Ma, Kim et al. 2003). Also, repair via non-homologous end joining (NHEJ) often results in small insertions or deletions and can be used for the creation of specific gene knockouts. Thus, one aspect of the present invention is to induce knock-outs or to introduce exogenous genetic sequences by homologous recombination into specific genetic loci.

By genetic sequence of interest is meant any endogenous nucleic acid sequence, such as, for example a gene or a non-coding sequence within or adjacent to a gene, in which it is desirable modify by targeted cleavage and/or targeted homologous recombination. The sequence of interest can be present in a chromosome, an episome, an organellar genome such as mitochondrial or chloroplast genome or genetic material that can exist independently to the main body of genetic material such as an infecting viral genome, plasmids, episomes, transposons for example. A sequence of interest can be within the coding sequence of a gene, within transcribed non-coding sequence such as, for example, leader sequences, trailer sequence or introns, or within non-transcribed sequence, either upstream or downstream of the coding sequence.

The first and the second double-stranded nucleic acid targets are comprised within the genetic sequence of interest into which it is desired to introduce a cleavage and thus genetic modification. Said modification may be a deletion of the genetic material, insertion of nucleotides in the genetic material or a combination of both deletion and insertion of nucleotides. By "target nucleic acid sequence", "double-stranded nucleic acid target" or "DNA target" is intended a polynucleotide that can be processed by the Cas9-tracrRNA:crRNA complex according to the present invention. The double-stranded nucleic acid target sequence is defined by the 5' to 3' sequence of one strand of said target. These terms refer to a specific DNA location within the genetic sequence of interest. The two targets can be spaced away each other from 1 to 500 nucleotides, preferably between 3 to 300 nucleotides, more preferably between 3 to 50 nucleotides, again more preferably between 1 to 20 nucleotides.

Any potential selected double-stranded DNA target in the present invention may have a specific sequence on its 3' end, named the protospacer adjacent motif or protospacer associated motif (PAM). The PAM is present in the strand of the nucleic acid target sequence which is not complementary to the crRNA. Preferably, the proto-spacer adjacent motif (PAM) may correspond to 2 to 5 nucleotides starting immediately or in the vicinity of the proto-spacer at the 3'-end. The sequence and the location of the PAM motif recognized by specific Cas9 vary among the different systems. PAM motif can be for examples NNAGAA, NAG, NGG, NGGNG, AWG, CC, CCN, TCN, TTC as non limiting examples (Shah, Erdmann et al. 2013). Different Type II systems have differing PAM requirements. For example, the *S. pyogenes* system requires an NGG sequence, where N can be any nucleotides. *S. thermophilus* Type II systems require NGGNG (Horvath and Barrangou 2010) and NNAGAAW (Deveau, Barrangou et al. 2008), while different *S. mutant* systems tolerate NGG or NAAR (van der Ploeg 2009). PAM is not restricted to the region adjacent to the proto-spacer but can also be part of the proto-spacer (Mojica, Diez-Villasenor et al. 2009). In a particular embodiment, the Cas9 protein can be engineered to recognize a non-natural PAM motif. In this case, the selected target sequence may comprise a smaller or a larger PAM motif with any combinations of amino acids. As non-limiting example, the two PAM motifs of the two nucleic acid targets can be present on the same nucleic acid strand and thus the Cas9 nickase harboring a non-functional RuvC or HNH nuclease domain induces two nick events on the same strand (Figures 2C and D). In this case, the resulting single-strand nucleic acid located between the first and the second nick can be deleted. This deletion may be repaired by NHEJ or homologous recombination mechanisms. In another aspect of the invention, the two PAM motifs of the two nucleic acid targets can be present on opposed nucleic acid strands and thus the Cas9 nickase harboring a non-functional RuvC or HNH nuclease domain induces two nick events on each strand of the genetic sequence of interest (Figures 2A and B) resulting in a double strand break within the genetic sequence of interest.

In a particular embodiment, the method of the present invention used two Cas9 nickases, each one capable of recognizing different PAM motifs within the two nucleic acid targets. As non-limiting example, the first Cas9 is capable of recognizing the NGG PAM motif and the second Cas9 is capable of recognizing the NNAGAAW PAM motif.

In particular, the present invention relates to a method comprising one or several of the following steps:
(a) selecting first and second double-stranded nucleic acid target sequences each comprising in one strand a PAM motif at its 3' extremity, wherein said PAM motifs are different;
(b) engineering two crRNAs comprising each a sequence complementary to a part of the other strand of the first and second double-stranded nucleic acid targets and having a 3' extension sequence;
(c) providing at least one tracrRNA comprising a sequence complementary to a part of the 3' extension sequences of said crRNAs;
(d) providing a first cas9 nuclease specifically recognizing the PAM motif of the first target and harboring a non-functional RuvC-like or HNH nuclease domain;
(e) providing a second Cas9 specifically recognizing the PAM motif of the second target and harboring a non-functional RuvC-like or HNH nuclease domain;
(f) introducing into the cell said crRNAs, said tracrRNA(s), said Cas9 nucleases such that each Cas9-tracrRNA:crRNA complex induces a nick event in the double-stranded nucleic acid target,
wherein the cell is a T-cell.

As non-limiting examples, *S. pyogenes* Cas9 lacking functional RuvC or HNH catalytic domain and S. *thermophilus* Cas9 lacking functional RuvC or HNH catalytic domain can be introduced into the cell to specifically recognize NGG PAM motif in the first target nucleic acid sequence and NNAGAAW PAM motif in the second target nucleic acid sequence respectively. In particular embodiment, the two distinct PAM motifs of the two nucleic acid targets can be present on the same nucleic acid strand and thus the Cas9 nickases harboring a non-functional RuvC or HNH nuclease domain induces two nick events on the same strand. In this case, the resulting single-strand nucleic acid located between the first and the second nick can be deleted. In another embodiment, the two distinct PAM motifs of the two nucleic acid targets can be present on opposed nucleic acid strands and thus the Cas9 nickases harboring a non-functional RuvC-like or HNH nuclease domain induces two nick events on each strand of the genetic sequence of interest resulting in a double-strand break within the genetic sequence of interest.

In another particular embodiment, the first Cas9 nickase harbors a non-functional RuvC-like nuclease domain and the second Cas9 nickase harbors a non-functional HNH nuclease domain. The different PAM motifs of the two nucleic acid targets can be on the same strand, thus the two Cas9 nickases induce a nick event on each strand (Figure 3A), resulting in a double-strand break within the genetic sequence of interest. The two PAM motifs can also be on opposed strands and thus the two Cas9 nickases induce a nick event on the same strand of the genetic sequence of interest (Figure 3B). In this case, the resulting single-strand nucleic acid located between the first and the second nick can be deleted. This deletion may be repaired by NHEJ or homologous recombination mechanisms.

The method of the present invention comprises engineering two crRNAs with distinct complementary regions to each nucleic acid target. In natural type II CRISPR system, the CRISPR targeting RNA (crRNA) targeting sequences are transcribed from DNA sequences known as protospacers. Protospacers are clustered in the bacterial genome in a group called a CRISPR array. The protospacers are short sequences of known foreign DNA separated by a short palindromic repeat and kept like a record against future encounters. To create the crRNA, the CRISPR array is transcribed and the RNA is processed to separate the individual recognition sequences between the repeats. The Spacer-containing CRISPR locus is transcribed in a long pre-crRNA. The processing of the CRISPR array transcript (pre-crRNA) into individual crRNAs is dependent on the presence of a trans-activating crRNA (tracrRNA) that has sequence complementary to the palindromic repeat. The tracrRNA hybridizes to the repeat regions separating the spacers of the pre-crRNA, initiating dsRNA cleavage by endogenous RNase III, which is followed by a second cleavage event within each spacer by Cas9, producing mature crRNAs that remain associated with the tracrRNA and Cas9 and form the Cas9-tracrRNA:crRNA complex. Engineered crRNA with tracrRNA is capable of targeting a selected nucleic acid sequence, obviating the need of RNase III and the crRNA processing in general (Jinek, Chylinski et al. 2012).

In the present invention, two crRNA are engineered to comprise distinct sequences complementary to a part of one strand of the two nucleic acid targets such that it is capable of targeting, preferably inducing a nick event in each nucleic acid targets. In particular embodiment, the two nucleic acid targets are spaced away each other from 1 to 300 bp, preferably from 3 to 250 bp, preferably from 3 to 200 bp, more preferably from 3 to 150 bp, 3 to 100bp, 3 to 50bp, 3 to 25 bp, 3 to 10 bp.

crRNA sequence is complementary to a strand of nucleic acid target, this strand does not comprise the PAM motif at the 3'-end (Figure 1). In a particular embodiment, each crRNA comprises a sequence of 5 to 50 nucleotides, preferably 8 to 20 nucleotides, more preferably 12 to 20 nucleotides which is complementary to the target nucleic acid sequence. In a more particular embodiment, the crRNA is a sequence of at least 30 nucleotides which comprises at least 10 nucleotides, preferably 12 nucleotides complementary to the target nucleic acid sequence. In particular, each crRNA may comprise a complementary sequence followed by 4-10 nucleotides on the 5'end to improve the efficiency of targeting (Cong, Ran et al. 2013; Mali, Yang et al. 2013; Qi, Larson et al. 2013). In preferred embodiment, the complementary sequence of the crRNA is followed in 3'-end by a nucleic acid sequences named repeat sequence or 3' extension sequence.

The crRNA according to the present invention can also be modified to increase its stability of the secondary structure and/or its binding affinity for Cas9. In a particular embodiment, the crRNA can comprise a 2', 3'-cyclic phosphate. The 2', 3'- cyclic phosphate terminus seems to be involved in many cellular processes i.e. tRNA splicing, endonucleolytic cleavage by several ribonucleases, in self-cleavage by RNA ribozyme and in response to various cellular stress including accumulation of unfolded protein in the endoplasmatic reticulum and oxidative stress (Schutz, Hesselberth et al. 2010). The inventors have speculated that the 2', 3'-cyclic phosphate enhances the crRNA stability or its affinity/specificity for Cas9. Thus, the present invention relates to the modified crRNA comprising a 2', 3'-cyclic phosphate, and the methods for genome engineering based on the CRISPR/cas system (Jinek, Chylinski et al. 2012; Cong, Ran et al. 2013; Mali, Yang et al. 2013) comprising using the modified crRNA.

In a particular embodiment, the crRNA can be engineered to recognize at least the two target nucleic acid sequences simultaneously. In this case, same crRNA comprises at least two sequences complementary to a portion of the two target nucleic acid sequences. In a preferred embodiment, said complementary sequences are spaced by a repeat sequence.

Trans-activating CRISPR RNA according to the present invention are characterized by an anti-repeat sequence capable of base-pairing with at least a part of the 3' extension sequence of crRNA to form a tracrRNA:crRNA also named guide RNA (gRNA). TracrRNA comprises a sequence complementary to a region of the crRNA.

A synthetic single guide RNA (sgRNA) comprising a fusion of crRNA and tracrRNA that forms a hairpin that mimics the tracrRNA-crRNA complex (Cong, Ran et al. 2013; Mali, Yang et al. 2013) can be used to direct Cas9 endonuclease-mediated cleavage of target nucleic acid. This system has been shown to function in a variety of eukaryotic cells, including human, zebra fish and yeast. The sgRNA may comprise two distinct sequences complementary to a portion of the two target nucleic acid sequences, preferably spaced by a repeat sequence.

The methods of the invention involve introducing crRNA, tracrRNA, sgRNA and Cas9 into a T-cell. crRNA, tracrRNA, sgRNA or Cas9 may be synthesized *in situ* in the cell as a result of the introduction of polynucleotide encoding RNA or polypeptides into the cell. Alternatively, the crRNA, tracRNA, sgRNA, Cas9 RNA or Cas9 polypeptides could be produced outside the cell and then introduced thereto. Methods for introducing a polynucleotide construct into animals are known in the art and including as non limiting examples stable transformation methods wherein the polynucleotide construct is integrated into the genome of the cell, transient transformation methods wherein the polynucleotide construct is not integrated into the genome of the cell and virus mediated methods. Said polynucleotides may be introduced into a cell by for example, recombinant viral vectors (e.g. retroviruses, adenoviruses), liposomes and the like. For example, transient transformation methods include for example microinjection, electroporation or particle bombardment. Said polynucleotides may be included in vectors, more particularly plasmids or virus, in view of being expressed in T-cells.

Also disclosed herein are polynucleotides, in particular DNA or RNA encoding the polypeptides and proteins previously described. These polynucleotides may be included in vectors, more particularly plasmids or virus, in view of being expressed in T-cells.

The present invention contemplates modification of the Cas9 polynucleotide sequence such that the codon usage is optimized for the organism in which it is being introduced. Thus, for example Cas9 polynucleotide sequence derived from the *pyogenes* or *S. Thermophilus* codon optimized for use in human is set forth in (Cong, Ran et al. 2013; Mali, Yang et al. 2013).

In particular embodiments, the Cas9 polynucleotides according to the present invention can comprise at least one subcellular localization motif. A subcellular localization motif refers to a sequence that facilitates transporting or confining a protein to a defined subcellular location that includes at least one of the nucleus, cytoplasm, plasma membrane, endoplasmic reticulum, golgi apparatus, endosomes, peroxisomes and mitochondria. Subcellular localization motifs are well-known in the art. A subcellular localization motif requires a specific orientation, e.g., N- and/or C-terminal to the protein. As a non-limiting example, the nuclear localization signal (NLS) of the simian virus 40 large T-antigen can be oriented at the N and/or C-terminus. NLS is an amino acid sequence which acts to target the protein to the cell nucleus through Nuclear Pore Complex and to direct a newly synthesized protein into the nucleus via its recognition by cytosolic nuclear transport receptors. Typically, a NLS consists of one or more short sequences of positively charged amino acids such as lysines or arginines.

The present invention also relates to a method for modifying genetic sequence of interest further comprising the step of expressing an additional catalytic domain into a host cell. In a more preferred embodiment, the present invention relates to a method to increase mutagenesis wherein said additional catalytic domain is a DNA end-processing enzyme. Non limiting examples of DNA end-processing enzymes include 5-3' exonucleases, 3-5' exonucleases, 5-3' alkaline exonucleases, 5' flap endonucleases, helicases, hosphatase, hydrolases and template-independent DNA polymerases. Non limiting examples of such catalytic domain comprise of a protein domain or catalytically active derivate of the protein domain seleced from the group consisting of hExol (EXO1_HUMAN), Yeast Exol (EXO1_YEAST), E.coli Exol, Human TREX2, Mouse TREX1, Human TREX1, Bovine TREX1, Rat TREX1, TdT (terminal deoxynucleotidyl transferase) Human DNA2, Yeast DNA2 (DNA2_YEAST). In a preferred embodiment, said additional catalytic domain has a 3'-5'-exonuclease activity, and in a more preferred embodiment, said additional catalytic domain has TREX exonuclease activity, more preferably TREX2 activity. In another preferred embodiment, said catalytic domain is encoded by a single chain TREX polypeptide.

Endonucleolytic breaks are known to stimulate the rate of homologous recombination. Therefore, in another preferred embodiment, the present invention relates to a method for inducing homologous gene targeting in the genetic sequence of interest further comprising providing to the cell an exogeneous nucleic acid comprising at least a sequence homologous to a portion of the genetic sequence of interest, such that homologous recombination occurs between the genetic sequence of interest and the exogenous nucleic acid.

In particular embodiments, said exogenous nucleic acid comprises first and second portions which are homologous to region 5' and 3' of the genetic sequence of interest respectively. Said exogenous nucleic acid in these embodiments also comprises a third portion positioned between the first and the second portion which comprises no homology with the regions 5' and 3' of the genetic sequence of interest. Following cleavage of the genetic sequence of interest, a homologous recombination event is stimulated between the target nucleic acid sequence and the exogenous nucleic acid. Preferably, homologous sequences of at least 50 bp, preferably more than 100 bp and more preferably more than 200 bp are used within said exogenous nucleic acid. Therefore, the exogenous nucleic acid is preferably from 200 bp to 6000 bp, more preferably from 1000 bp to 2000 bp. Indeed, shared nucleic acid homologies are located in regions flanking upstream and downstream the cleavage induced and the nucleic acid sequence to be introduced should be located between the two arms.

Depending on the location of the genetic sequence of interest wherein break event has occurred, such exogenous nucleic acid can be used to knock-out a gene, e.g. when exogenous nucleic acid is located within the open reading frame of said gene, or to introduce new sequences or genes of interest. Sequence insertions by using such exogenous nucleic acid can be used to modify a targeted existing gene, by correction or replacement of said gene (allele swap as a non-limiting example), or to up- or down-regulate the expression of the targeted gene (promoter swap as non-limiting example), said targeted gene correction or replacement.

### Modified cells and kits

T-cells are used in the method according to the invention. T-cells can be cell lines for *in vitro* cultures or primary cells of animal origin.

By "primary cell" or "primary cells" are intended cells taken directly from living tissue (i.e. biopsy material) and established for growth in vitro, that have undergone very few population doublings and are therefore more representative of the main functional components and characteristics of tissues from which they are derived from, in comparison to continuous tumorigenic or artificially immortalized cell lines. These cells thus represent a more valuable model to the *in vivo* state they refer to.

In the frame of the present invention, "T-cells" refer to an animal T-cell or a T-cell line derived from the organisms listed below and established for in vitro culture.

More preferably the animal cell is of the genus *Homo, Rattus, Mus, Sus, Bos, Danio, Canis, Felis, Equus, Salmo, Oncorhynchus, Gallus, Meleagris,* more preferably, the animal cell is of the species *Homo sapiens, Rattus norvegicus, Mus musculus, Sus scrofa, Bos taurus, Danio rerio, Canis lupus, Felis catus, Equus caballus, Salmo salar, Oncorhynchus mykiss, Gallus gallus, Meleagris gallopavo.*

In the present invention, the T-cell is preferably a mammalian cell or cell line for *in vitro* cultures or primary cells taken directly from living tissue and established for *in vitro* culture. As non limiting examples cell lines can be selected from the group consisting of Jurkat cells and Molt 4 cells.

These cell lines can be modified by the method of the present invention to provide cell line models to produce, express, quantify, detect, study a gene or a protein of interest; these models can also be used to screen biologically active molecules of interest in research and production and various fields such as chemical, biofuels, therapeutics and agronomy as non-limiting examples. A particular aspect of the present invention relates to an isolated cell as previously described obtained by the method according to the invention. Typically, said isolated cell comprises Cas9 nickases, crRNA(s) and tracrRNA or sgRNA. Resulting isolated cell comprises a modified genetic sequence of interest in which a cleavage has occurred. The resulting modified cell can be used as a cell line for a diversity of applications such as cell therapy by using T-cells. The methods of the invention are useful to engineer genomes and to reprogram cells. Also disclosed herein is a kit for cell transformation comprising one or several of the components of the modified type II CRISPR system according to the invention as previously described. This kit more particularly comprises:
- two crRNAs comprising a sequence complementary to one strand of a first and second double-strand nucleic acid target sequences comprising PAM motif in the other strand and having a 3' extension sequence ;
- at least one tracrRNA comprising a sequence complementary to the 3' extension sequences of said crRNAs;
- at least one cas9 nuclease harboring a non-functional RuvC-like or HNH nuclease domain or a polynucleotide encoding thereof.

In another embodiment, the kit comprises:
- Two crRNAs comprising a sequence complementary to one strand of a first and second double-strand nucleic acid target sequences comprising different PAM motifs in the other strand and having a 3'extension sequence;
- at least one tracrRNA comprising a sequence complementary to the 3' extension sequences of said crRNAs;
- a first Cas9 nuclease specifically recognizing the PAM motif of the first nucleic acid target and harboring a non-functional RuvC-like or a polynucleotide encoding thereof.
- a second Cas9 nuclease specifically recognizing the PAM motif of the second nucleic acid target and harboring a non-functional HNH nuclease domain or a polynucleotide encoding thereof.

### Therapeutic applications

The method disclosed herein can have a variety of applications. In one embodiment, the method can be used for clinical or therapeutic applications. The method can be used to repair or correct disease-causing genes. The method can be used to correct splice junction mutations, deletions, insertions, and the like in other genes or chromosomal sequences that play a role in a particular disease or disease state.

Such methods can also be used to genetically modify primary T-cells, with a view to injecting such cells into a patient for treating a disease or infection. Such cell therapy schemes are more particularly developed for treating cancer, viral infection such as caused by CMV or HIV or self-immune diseases.

### Definitions

In the description above, a number of terms are used extensively. The following definitions are provided to facilitate understanding of the present embodiments.

As used herein, "a" or "an" may mean one or more than one.

Amino acid residues in a polypeptide sequence are designated herein according to the one-letter code, in which, for example, Q means Gin or Glutamine residue, R means Arg or Arginine residue and D means Asp or Aspartic acid residue.

Amino acid substitution means the replacement of one amino acid residue with another, for instance the replacement of an Arginine residue with a Glutamine residue in a peptide sequence is an amino acid substitution.

Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.

As used herein, "nucleic acid" or polynucleotide" refers to nucleotides and/or polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Nucleic acids can be either single stranded or double stranded.

By "complementary sequence" is meant the sequence part of polynucleotide (e.g. part of crRNa or tracRNA) that can hybridize to another part of polynucleotides (e.g. the target nucleic acid sequence or the crRNA respectively) under standard low stringent conditions. Such conditions can be for instance at room temperature for 2 hours by using a buffer containing 25% formamide, 4x SSC, 50 mM NaH2PO4/Na2HPO4 buffer; pH 7.0,5x Denhardt's, 1 mM EDTA,1 mg/ml DNA + 20 to 200 ng/ml probe to be tested (approx. 20 - 200 ng/ml)). This can be also predicted by standard calculation of hybridization using the number of complementary bases within the sequence and the content in G-C at room temperature as provided in the literature. Preferentially, the sequences are complementary to each other pursuant to the complementarity between two nucleic acid strands relying on Watson-Crick base pairing between the strands, i.e. the inherent base pairing between adenine and thymine (A-T) nucleotides and guanine and cytosine (G-C) nucleotides. Accurate base pairing equates with Watson-Crick base pairing includes base pairing between standard and modified nucleosides and base pairing between modified nucleosides, where the modified nucleosides are capable of substituting for the appropriate standard nucleosides according to the Watson-Crick pairing. The complementary sequence of the single-strand oligonucleotide can be any length that supports specific and stable hybridization between the two single-strand oligonucleotides under the reaction conditions. The complementary sequence generally authorizes a partial double stranded overlap between the two hybridized oligonucleotides over more than 3bp, preferably more than 5 bp, preferably more than to 10 bp. The complementary sequence is advantageously selected not to be homologous to any sequence in the genome to avoid off-target recombination or recombination not involving the whole exogenous nucleic acid sequence (i.e. only one oligonucleotide).

By "nucleic acid homologous sequence" it is meant a nucleic acid sequence with enough identity to another one to lead to homologous recombination between sequences, more particularly having at least 80% identity, preferably at least 90% identity and more preferably at least 95%, and even more preferably 98 % identity. "Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting.

The terms "vector" or "vectors" refer to a nucleic acid molecule capable of transporting another nucleic acid to which it has been linked. A "vector" in the present invention includes, but is not limited to, a viral vector, a plasmid, a RNA vector or a linear or circular DNA or RNA molecule which may consists of a chromosomal, non-chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available. Viral vectors include retrovirus, adenovirus, parvovirus (e. g. adenoassociated viruses), coronavirus, negative strand RNA viruses such as orthomyxovirus (e. g., influenza virus), rhabdovirus (e. g., rabies and vesicular stomatitis virus), paramyxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).

The present invention is illustrated by reference to certain specific examples.

### REFERENCES

Chylinski, K., A. Le Rhun, et al. (2013). "The tracrRNA and Cas9 families of type II CRISPR-Cas immunity systems." RNA Biol 10(5).
Cong, L., F. A. Ran, et al. (2013). "Multiplex genome engineering using CRISPR/Cas systems." Science 339(6121): 819-23.
Critchlow, S. E. and S. P. Jackson (1998). "DNA end-joining: from yeast to man." Trends Biochem Sci 23(10): 394-8.
Dalgaard, J. Z., A. J. Klar, et al. (1997). "Statistical modeling and analysis of the LAGLIDADG family of site-specific endonucleases and identification of an intein that encodes a site-specific endonuclease of the HNH family." Nucleic Acids Res 25(22): 4626-38.
Deltcheva, E., K. Chylinski, et al. (2011). "CRISPR RNA maturation by trans-encoded small RNA and host factor RNase III." Nature 471(7340): 602-7.
Deveau, H., R. Barrangou, et al. (2008). "Phage response to CRISPR-encoded resistance in Streptococcus thermophilus." J Bacteriol 190(4): 1390-400.
Garneau, J. E., M. E. Dupuis, et al. (2010). "The CRISPR/Cas bacterial immune system cleaves bacteriophage and plasmid DNA." Nature 468(7320): 67-71.
Gasiunas, G., R. Barrangou, et al. (2012). "Cas9-crRNA ribonucleoprotein complex mediates specific DNA cleavage for adaptive immunity in bacteria." Proc Natl Acad Sci U S A 109(39): E2579-86.
Gorbalenya, A. E. (1994). "Self-splicing group I and group II introns encode homologous (putative) DNA endonucleases of a new family." Protein Sci 3(7): 1117-20.
Haft, D. H., J. Selengut, et al. (2005). "A guild of 45 CRISPR-associated (Cas) protein families and multiple CRISPR/Cas subtypes exist in prokaryotic genomes." PLoS Comput Biol 1(6): e60.
Horvath, P. and R. Barrangou (2010). "CRISPR/Cas, the immune system of bacteria and archaea." Science 327(5962): 167-70.
Jiang, W., D. Bikard, et al. (2013). "RNA-guided editing of bacterial genomes using CRISPR-Cas systems." Nat Biotechnol 31(3): 233-9.
Jinek, M., K. Chylinski, et al. (2012). "A programmable dual-RNA-guided DNA endonuclease in adaptive bacterial immunity." Science 337(6096): 816-21.
Kleanthous, C., U. C. Kuhlmann, et al. (1999). "Structural and mechanistic basis of immunity toward endonuclease colicins." Nat Struct Biol 6(3): 243-52.
Ma, J. L., E. M. Kim, et al. (2003). "Yeast Mre11 and Rad1 proteins define a Ku-independent mechanism to repair double-strand breaks lacking overlapping end sequences." Mol Cell Biol 23(23): 8820-8.
Makarova, K. S., N. V. Grishin, et al. (2006). "A putative RNA-interference-based immune system in prokaryotes: computational analysis of the predicted enzymatic machinery, functional analogies with eukaryotic RNAi, and hypothetical mechanisms of action." Biol Direct 1: 7.
Mali, P., L. Yang, et al. (2013). "RNA-guided human genome engineering via Cas9." Science 339(6121): 823-6.
Mojica, F. J., C. Diez-Villasenor, et al. (2009). "Short motif sequences determine the targets of the prokaryotic CRISPR defence system." Microbiology 155(Pt 3): 733-40.
Qi, L. S., M. H. Larson, et al. (2013). "Repurposing CRISPR as an RNA-guided platform for sequence-specific control of gene expression." Cell 152(5): 1173-83.
Sapranauskas, R., G. Gasiunas, et al. (2011). "The Streptococcus thermophilus CRISPR/Cas system provides immunity in Escherichia coli." Nucleic Acids Res 39(21): 9275-82.
Schutz, K., J. R. Hesselberth, et al. (2010). "Capture and sequence analysis of RNAs with terminal 2',3'-cyclic phosphates." Rna 16(3): 621-31.
Shah, S. A., S. Erdmann, et al. (2013). "Protospacer recognition motifs: Mixed identities and functional diversity." RNA Biol 10(5).
Shub, D. A., H. Goodrich-Blair, et al. (1994). "Amino acid sequence motif of group I intron endonucleases is conserved in open reading frames of group II introns." Trends Biochem Sci 19(10): 402-4.
Sorek, R., C. M. Lawrence, et al. (2013). "CRISPR-mediated Adaptive Immune Systems in Bacteria and Archaea." Annu Rev Biochem.
van der Ploeg, J. R. (2009). "Analysis of CRISPR in Streptococcus mutans suggests frequent occurrence of acquired immunity against infection by M102-like bacteriophages." Microbiology 155(Pt 6): 1966-76.

### SEQUENCE LISTING

<110> Cellectis
<120> A METHOD FOR PRODUCING PRECISE DNA CLEAVAGE USING CAS9 NICKASE
   ACTIVITY
<130> P81303480PCT00
<150> PA201370295
   <151> 2013-05-29
<160> 1
<170> PatentIn version 3.5
<210> 1
   <211> 1368
   <212> PRT
   <213> Streptococcus pyogenes serotype M1
<220>
   <223> Cas9
<400> 1

## Claims

1. An in vitro method for precisely inducing a nucleic acid cleavage in a genetic sequence in a cell comprising:
(a) Selecting a first and second double-stranded nucleic acid target in said genetic sequence, each nucleic acid target comprising, on one strand, a protospacer adjacent motif (PAM) at one 3' extremity;
(b) engineering two CRISPR targeting RNAs (crRNAs) comprising each:
- a sequence complementary to one part of the opposite strand of the nucleic acid target that does not comprise the PAM motif, and
- a 3' extension sequence ;
(c) providing at least one trans-activating CRISPR targeting RNA (tracrRNA) comprising a sequence complementary to one part of the 3' extension sequences of said crRNAs under b);
(d) providing at least one cas9 nickase which is a cas9 nuclease harboring either a non-functional RuvC-like or a non-functional HNH nuclease domain and recognizing said PAM motif(s);
(e) introducing into the cell said crRNAs, said tracrRNA(s) and said Cas9 nickase; wherein the cell is a T-cell,
such that each Cas9-tracrRNA:crRNA complex induces a nick event in double-stranded nucleic acid targets in order to cleave the genetic sequence between said first and second nucleic acid targets.

2. The method of claim 1, wherein the two PAM motifs are present on opposed nucleic acid strands.

3. The method of claim 1, wherein the two PAM motifs are present on the same nucleic acid strand.

4. The method according to any one of claims 1 to 3 wherein the first and second double-stranded nucleic acid targets comprise different PAM motifs specifically recognized by two different Cas9 nickases.

5. The method of claim 4, wherein said method involves a first Cas9 nickase harboring a non-functional RuvC-like and a second Cas9 nickase harboring a non-functional HNH nuclease domain.

6. The method according to claims 1 to 5, wherein at least one Cas9 nickase comprises at least one mutation in the RuvC domain.

7. The method according to claims 1 to 5, wherein at least one Cas 9 nickase comprises at least one mutation in the HNH domain.

8. The method according to any one of claims 1 to 7, wherein each crRNA comprises complementary sequence from 12 to 20 nucleotides.

9. The method according to any one of claims 1 to 8, comprising in step b) engineering one crRNA comprising two sequences complementary to a part of each target nucleic acid sequences.

10. The method according to any one of claims 1 to 9, wherein the crRNA and the tracrRNA are fused to form a single guide RNA.

11. The method according to any one of claims 1 to 10, wherein the first and the second nucleic acid target sequences are spaced from each other by a spacer region from 1 to 300 bp, preferably from 3 to 250 bp.

12. The method according to any one of claims 1 to 11, further comprising introducing an exogenous nucleic acid sequence comprising at least one sequence homologous to at least a portion of the genetic sequence, such that homologous recombination occurs between said exogenous sequence and genetic sequence.

13. The method according to any one of claims 1 to 12, wherein said T-cell is a primary T-cell.

14. An isolated T- cell comprising:
- two crRNAs comprising sequences complementary to a first and second double-strand nucleic acid target sequences and having a 3' extension sequence ;
- at least one tracrRNA comprising a sequence complementary to the 3' extension sequences of said crRNAs;
- at least one cas9 nickase which is a cas9 nuclease harboring either a non-functional RuvC-like domain or a non-functional HNH nuclease domain or a polynucleotide encoding thereof.

15. The T-cell according to claim 14 for use in treating cancer, viral infection or self-immune diseases.

16. The T-cell for use according to claim 15 wherein the T-cell is a primary T-cell.

## Patentansprüche

1. In vitro-Verfahren zum präzisen Induzieren einer Nukleinsäurespaltung in einer Gensequenz in einer Zelle, umfassend:
(a) Auswahl eines ersten und zweiten doppelsträngigen Nukleinsäure-Targets in der genannten genetischen Sequenz, wobei jedes Nukleinsäure-Target an einem Strang ein Protospacer Adjacent Motif (PAM) an einem 3'-Ende umfasst;
(b) Entwickeln von zwei CRISPR-Targeting RNAs (crRNAs), jeweils umfassend:
- eine komplementäre Sequenz zu einem Teil des entgegengesetzten Strangs des Nukleinsäure-Targets, die das PAM-Motif nicht umfasst, und
- eine 3'-Erweiterungssequenz;
(c) Bereitstellen von wenigstens einer transaktivierenden CRISPR-Targeting RNA (tracrRNA), umfassend eine Sequenz, die zu einem Teil der 3'-Erweiterungssequenzen der genannten crRNAs unter b) komplementär sind;
(d) Bereitstellen wenigstens einer cas9-Nickase, die eine cas9-Nuklease ist, die entweder eine nicht funktionale RuvC-ähnliche oder eine nicht funktionale HNH-Nukleasendomäne ist und das / die genannte (n) PAM-Motiv(e) erkennt;
(e) Einfügen der genannten crRNAs, der genannten tracrRNA(s) und der genannten Cas9-Nickase in die Zelle, wobei die Zelle eine T-Zelle ist,
derart, dass jeder Cas9-tracrRNA: crRNA-Komplex ein Kerbenvorkommen in doppelsträngigen Nukleinsäure-Targets induziert, um die genetische Sequenz zwischen den genannten ersten und zweiten Nukleinsäure-Targets zu spalten.

2. Verfahren gemäß Anspruch 1, wobei die zwei PAM-Motive auf entgegengesetzten Nukleinsäuresträngen vorhanden sind.

3. Verfahren gemäß Anspruch 1, wobei die zwei PAM-Motive auf demselben Nukleinsäurestrang vorhanden sind.

4. Verfahren gemäß irgendeinem der Ansprüche 1 bis 3, wobei das erste und zweite doppelsträngige Nukleinsäure-Targets unterschiedliche PAM-Motive umfassen, die spezifisch von zwei unterschiedlichen Cas9-Nickasen erkannt werden.

5. Verfahren gemäß Anspruch 4, wobei das genannte Verfahren eine erste Cas9-Nickase umfasst, die eine nicht funktionale RuvC-ähnliche und eine zweite Cas9-Nickase impliziert, die eine nicht funktionale HNH-Nukleasendomäne verbirgt.

6. Verfahren gemäß Anspruch 1 bis 5, wobei wenigstens eine Cas9-Nickase wenigstens eine Mutation in der RuvC-Domäne umfasst.

7. Verfahren gemäß Anspruch 1 bis 5, wobei die wenigstens eine Cas9-Nickase wenigstens eine Mutation in der HNH-Domäne umfasst.

8. Verfahren gemäß irgendeinem der Ansprüche 1 bis 7, wobei jede crRNA eine komplementäre Sequenz von 12 bis 20 Nukleotiden umfasst.

9. Verfahren gemäß irgendeinem der Ansprüche 1 bis 8, umfassend in Schritt b) das Entwickeln einer crRNA, umfassend zwei komplementäre Sequenzen zu einem Teil jeder der Target-Nukleinsäuresequenzen.

10. Verfahren gemäß irgendeinem der Ansprüche 1 bis 9, wobei die crRNA und die tracrRNA zusammengebunden sind, um eine einzige Führungs-RNA zu bilden.

11. Verfahren gemäß irgendeinem der Ansprüche 1 bis 10, wobei die erste und die zweite Target-Nukleinsäuresequenzen durch eine Beabstandungsregion von 1 bis 300 bp, bevorzugt von 3 bis 250 bp, beabstandet sind.

12. Verfahren gemäß irgendeinem der Ansprüche 1 bis 11, weiterhin umfassend das Einführen einer exogenen Nukleinsäuresequenz, umfassend wenigstens eine Sequenz, die homolog zu wenigstens einem Abschnitt der Gensequenz derart ist, dass eine homologe Rekombination zwischen der genannten exogenen Sequenz und der Gensequenz auftritt.

13. Verfahren gemäß irgendeinem der Ansprüche 1 bis 12, wobei die genannte T-Zelle eine primäre T-Zelle ist.

14. Isolierte T-Zelle, umfassend:
- zwei crRNAs, umfassend Sequenzen, die zu einer ersten und zweiten doppelsträngigen Nukleinsäure-Targetsequenz komplementär sind und eine 3'-Erweiterungssequenz haben;
- wenigsten eine tracrRNA, die eine Sequenz umfasst, die zu den 3`-Erweiterungssequenzen der genannten crRNAs komplementär ist;
- wenigstens eine cas9-Nickase, die eine cas9-Nuklease ist, die entweder eine nicht funktionale RuvC-ähnliche Domäne oder eine nicht funktionale HNH-Nukleasendomäne verbirgt, oder ein dafür kodierendes Polynukleotid.

15. T-Zelle gemäß Anspruch 14 für die Verwendung bei der Behandlung von Krebs, viralen Infektionen oder Auto-Immunerkrankungen.

16. T-Zelle gemäß Anspruch 15, wobei die T-Zelle eine primäre T-Zelle ist.

## Revendications

1. Procédé in vitro pour induire avec précision le clivage d'un acide nucléique dans une séquence génétique d'une cellule comprenant :
(a) la sélection d'un premier et d'un second acides nucléiques cibles double brin dans ladite séquence génétique, chaque acide nucléique cible comprenant, sur un brin, un motif adjacent au proto-espaceur (PAM) à une extrémité 3' ;
(b) l'ingénierie de deux ARN de ciblage CRISPR (crRNA) comprenant chacun :
- une séquence complémentaire à une partie du brin opposé de l'acide nucléique cible qui ne comprend pas le motif PAM, et
- une séquence d'extension 3' ;
(c) la fourniture d'au moins un ARN de ciblage CRISPR d'activation trans (tracrRNA) comprenant une séquence complémentaire à une partie des séquences d'extension 3' desdits crRNA selon b) ;
(d) la fourniture d'au moins une cas9 nickase qui est une cas9 nucléase comprenant soit un domaine nucléase HNH non fonctionnel soit un domaine RuvC non fonctionnel et reconnaissant ledit (lesdits) motif(s) PAM ;
(e) l'introduction dans ladite cellule desdits crRNA, dudit (desdits) tracrRNA et de ladite Cas9 nickase ;
dans lequel la cellule est un lymphocyte T,
de telle sorte que chaque complexe Cas9-tracrRNA : crRNA induit un événement de coupure dans les acides nucléiques cibles double brin, de manière à cliver la séquence génétique entre lesdits premier et second acides nucléiques cibles.

2. Procédé de la revendication 1, dans lequel les deux motifs PAM sont présents sur des brins d'acides nucléiques opposés.

3. Procédé de la revendication 1, dans lequel les deux motifs PAM sont présents sur le même brin d'acide nucléique.

4. Procédé selon l'une quelconque des revendications 1 à 3, dans lequel le premier et le second acides nucléiques cibles double brin comprennent différents motifs PAM spécifiquement reconnus par deux Cas9 nickases différentes.

5. Procédé de la revendication 4, dans lequel ledit procédé consiste en une première Cas9 nickase comprenant un domaine RuvC non fonctionnel et en une seconde Cas9 nickase comprenant un domaine nucléase HNH non fonctionnel.

6. Procédé selon les revendications 1 à 5, dans lequel au moins une Cas9 nickase comprend au moins une mutation dans le domaine RuvC.

7. Procédé selon les revendications 1 à 5, dans lequel au moins une Cas9 nickase comprend au moins une mutation dans le domaine HNH.

8. Procédé selon l'une quelconque des revendications 1 à 7, dans lequel chaque crRNA comprend une séquence complémentaire de 12 à 20 nucléotides.

9. Procédé selon l'une quelconque des revendications 1 à 8, comprenant à l'étape b) l'ingénierie d'un crRNA comprenant deux séquences complémentaires à une partie de chaque séquence d'acides nucléiques cibles.

10. Procédé selon l'une quelconque des revendications 1 à 9, dans lequel le crRNA et le tracrRNA sont fusionnés pour former un ARN guide unique.

11. Procédé selon l'une quelconque des revendications 1 à 10, dans lequel les première et seconde séquences d'acides nucléiques cibles sont espacées l'une de l'autre par une région intercalaire de 1 à 300 pb, de préférence, de 3 à 250 pb.

12. Procédé selon l'une quelconque des revendications 1 à 11, comprenant, en outre, l'introduction d'une séquence d'acides nucléiques exogène comprenant au moins une séquence homologue à au moins une portion de la séquence génétique, de telle sorte qu'une recombinaison homologue se produit entre lesdites séquence exogène et séquence génétique.

13. Procédé selon l'une quelconque des revendications 1 à 12, dans lequel ledit lymphocyte T est un lymphocyte T primaire.

14. Lymphocyte T isolé comprenant :
- deux crRNA comprenant des séquences complémentaires à une première et une seconde séquences d'acides nucléiques cibles double brin, ayant une séquence d'extension 3' :
- au moins un tracrRNA comprenant une séquence complémentaire aux séquences d'extension 3' desdits crRNA ;
- au moins une cas9 nickase qui est une cas9 nucléase comprenant soit un domaine nucléase HNH non fonctionnel soit un domaine RuvC non fonctionnel ou un polynucléotide codant pour celle-ci.

15. Lymphocyte T selon la revendication 14 destiné à être utilisé dans le traitement du cancer, d'une infection virale ou des maladies auto-immunes.

16. Lymphocyte T destiné à être utilisé selon la revendication 15, dans lequel le lymphocyte T est un lymphocyte T primaire.
